# FASCICULE DE BREVET EUROPEEN

(11) **EP 1 220 644 B1**
(45) Date de publication et mention de la délivrance du brevet: **29.12.2004**
(21) Numéro de dépôt: 00968041.4
(22) Date de dépôt: 13.10.2000
(51) Int. Cl.: A61B 17/70

(54) **IMPLANT INTERVERTEBRAL**
ZWISCHENWIRBELIMPLANTAT
INTERVERTEBRAL IMPLANT

(30) Priorité: 15.10.1999 FR 9913156
(43) Date de publication de la demande: 10.07.2002
(73) Titulaire: Spine Next, La Cité Mondiale, 33000 Bordeaux (FR)
(72) Inventeur: SENEGAS, Jacques, F-33700 Merignac (FR)
(74) Mandataire: Dronne, Guy
(86) Numéro de dépôt international: PCT/FR2000/002862
(87) Numéro de publication internationale: WO 2001/028442

(56) Documents cités:
- FR-A- 2 717 675
- FR-A- 2 775 183
- US-A- 5 496 318

## Description

La présente invention concerne un implant intervertébral comprenant une cale et un lien pour maintenir ladite cale entre les vertèbres.

Cette cale supplée le disque intervertébral lorsque celui-ci est défaillant et en particulier elle limite le rapprochement de la partie postérieure de deux vertèbres lors de l'extension du rachis. En effet, le rapprochement de la partie postérieure de deux vertèbres provoque des désagréments, et en particulier des douleurs.

En effet, lorsque le disque intervertébral est dégénéré, les mouvements du rachis peuvent provoquer des contacts intervertébraux susceptibles de pincer les racines nerveuses qui débouchent latéralement entre les vertèbres.

Pour remédier à cet inconvénient, il est connu de fixer au moins une cale entre deux apophyses épineuses de deux vertèbres consécutives. Ces apophyses épineuses prolongent les vertèbres sous forme d'épine dans la partie postérieure de la colonne vertébrale.

Ainsi, en interposant une cale entre deux apophyses successives, on évite le contact entre deux vertèbres lorsque le disque intervertébral fait défaut. FR-N-2 717 675 montre les caractéristiques du préambule de la revendication 1.

Cependant, cette cale doit être fixée aux vertèbres de façon suffisamment rigide pour garder sa position quels que soient les mouvements du rachis, et elle doit être suffisamment libre par rapport aux mêmes vertèbres pour ne pas rigidifier d'une façon trop importante la colonne vertébrale. II est connu de fixer ladite cale, dans laquelle sont ménagés un ou des perçages transversaux, au moyen de ligaments insérés dans ces perçages et formant des boucles dans lesquelles s'engagent les apophyses. Ce système de fixation est peu rigide et il nécessite un grand nombre de manipulations, ce qui augmente d'autant le temps de l'intervention chirurgicale.

Un objet de la présente invention est de fournir un implant intervertébral dont les moyens de fixation sur les apophyses épineuses sont susceptibles d'être mis en oeuvre dans un temps relativement court par rapport à la technique antérieure, et dont la fixation est plus rigide.

Pour atteindre ce but, conformément à l'invention l'implant intervertébral selon la revendication 1 comprend une cale dans laquelle sont ménagées deux gorges opposées susceptibles de recevoir les deux apophyses épineuses de deux vertèbres, chaque gorge définissant deux ailes ayant une paroi interne, et un lien pour maintenir ladite cale sur lesdites apophyses épineuses,
- ledit lien étant constitué d'au moins une bande dont une partie peut entourer une portion de surface de l'apophyse opposée au fond de la gorge,
ledit implant comprenant en outre
- des moyens de fixation, ménagés dans au moins une aile, pour fixer une première extrémité de ladite bande, et
caractérisé en ce que ledit implant comprend:
- des moyens de fixation auto-bloquant, ménagés dans au moins une autre aile, au travers desquels la deuxième extrémité de ladite bande peut être insérée puis tirée pour maintenir en position ladite bande par quoi ladite cale est solidaire desdites apophyses épineuses.

On comprend que la cale s'insère entre deux vertèbres et que chacune des apophyses qui les prolongent prend appui dans les gorges opposées de ladite cale. Les diverses flexions du rachis provoquent une certaine mobilité d'une apophyse par rapport à l'autre et le maintien en position de la cale est assuré par une bande qui assure une surface de contact avec l'apophyse plus importante que ne l'assure un lien du type ligament.

En conséquence le lien est plus solidement accroché sur l'apophyse. Par ailleurs, une extrémité de la bande est fixée dans une des deux ailes constituant la gorge, et avantageusement les moyens de fixation comprennent une fente, percée dans ladite aile, dans laquelle ladite première extrémité de ladite bande est susceptible d'être engagée de manière à former une boucle, par quoi ladite première extrémité de ladite bande est solidaire de ladite aile. Ce mode de fixation est relativement aisé à mettre en oeuvre. Ainsi l'extrémité de la bande est fixée à ladite cale par ligature du bout de la bande sur une portion de son extrémité après passage dudit bout dans ladite fente et formation de la boucle. Cette opération est réalisée en préalable à l'installation de la cale entre deux vertèbres.

La seconde extrémité de la bande est fixée sur ladite autre aile aux moyens de fixation auto-bloquant comprenant préférentiellement une première fente et une deuxième fente parallèles entre elles, percées dans ladite autre aile, ladite deuxième fente étant située entre ladite première fente et le fond de la gorge, de sorte que la portion de bande qui débouche de la première fente sur ladite paroi interne de ladite autre aile est appliquée contre la portion de bande qui pénètre dans la deuxième fente, par quoi l'extrémité de ladite bande est susceptible d'être bloquée par frottement.

De façon avantageuse ledit lien comporte deux bandes et ainsi chaque gorge comprend des moyens de fixation dans l'une de ses ailes et des moyens de fixations auto-bloquant ménagés dans l'autre aile, de manière à fixer deux bandes, chacune étant apte à entourer chacune des apophyses, par quoi lesdites apophyses sont enserrées indépendamment l'une de l'autre.

On comprend que les bandes sont susceptibles d'être pré-montées pour former une boucle sur chaque gorge. Le bout de la deuxième extrémité de chaque bande est engagé dans chaque deuxième fente sur la paroi interne de chacune desdites autres ailes et réintroduit dans lesdites premières fentes pour déboucher sur la paroi interne de chaque aile.

Lorsque la cale est insérée entre les vertèbres, les apophyses traversent les boucles, et la fixation complète est réalisée en tirant sur chaque bout de bande de façon à serrer lesdites apophyses dans les gorges.

On comprend également que les moyens auro-bloquants sont mis en oeuvre lors de l'opération de serrage. En effet, plus la bande est serrée sur l'apophyse, plus la portion de bande qui débouche sur la paroi interne de l'aile est pressée sur la portion de bande qui pénètre dans la deuxième fente. Ainsi, les deux portions de bandes sont pincées entre la paroi interne de l'aile et le corps de l'apophyse immobilisant ladite extrémité de bande par rapport à l'aile.

Selon un premier mode de réalisation la cale est fixée, en parallèle, sur les apophyses épineuses au moyen de deux bandes séparées.

Selon un deuxième mode de réalisation, la cale est fixée sur les apophyses épineuses au moyen d'une seule bande.

D'autres particularités et avantages de l'invention ressortiront à la lecture de la description faite ci-après, à titre indicatif mais non limitatif, en référence aux dessins annexés sur lesquels :
- la Figure 1 est une vue en perspective d'un premier mode de réalisation de l'implant intervertébral montrant une cale munie de deux bandes fixant la cale sur deux apophyses épineuses consécutives,
- la Figure 2 est une vue de l'implant intervertébral en coupe selon le plan A-A de la Figure 1 montrant la position des apophyses épineuses par rapport à la cale,
- la Figure 3 est une vue en perspective d'un deuxième mode de réalisation de l'implant intervertébral muni d'une seule bande ; et
- la Figure 4 est une vue en perspective d'un troisième mode de réalisation de l'implant intervertébral muni d'une seule bande.

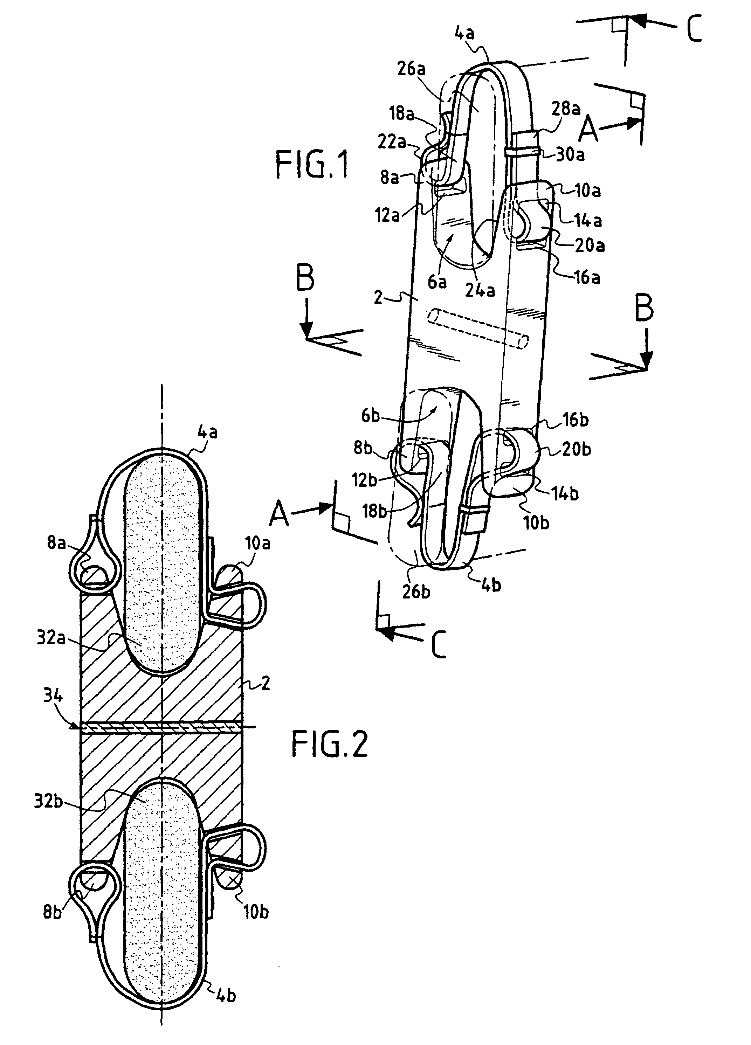

En se référant tout d'abord à la Figure 1, on va décrire un premier mode de réalisation de l'implant intervertébral dans le cas où le lien est constitué de deux bandes.

L'implant comprend une cale 2 et deux bandes 4a et 4b. La cale 2 a une forme générale de parallélépipède rectangle, dont les deux extrémités comportent chacune une gorge 6a et 6b. Ces gorges sont opposées ; elles sont symétriques par rapport au plan de symétrie B de la cale perpendiculaire aux plus grands côtés du parallélépipède et leurs plans de symétrie C sont confondus. Par ailleurs ces gorges font apparaître des ailes 8a, 10a, 8b, 10b, dans lesquelles sont ménagés des moyens de fixation 12a, 12b, 14a, 14b, 16a, 16b pour fixer les extrémités 18a, 18b, 20a, 20b des bandes.

Les moyens de fixation 12a et 12b sont identiques et sont respectivement ménagés dans les ailes 8a et 8b. En conséquence, on va décrire la fixation de la première extrémité 18a de la bande 4a sur l'aile 6a uniquement.

Le moyen de fixation 12a est constitué d'un percement de part en part de l'aile 8a, ce percement ayant la forme d'une fente dont les dimensions sont au moins égales à la section droite de la bande 4a de sorte qu'elle puisse y être introduite. Cette fente est sensiblement parallèle au plan de symétrie C des gorges et au plan de symétrie B de la cale.

Une portion 22a de l'extrémité 18a de la bande 4a est insérée au travers de l'aile 8a dans la fente 12a, puis rabattue sur une autre portion de l'extrémité de la bande pour former une boucle. Le bout de l'extrémité de la bande 4a est ligaturé sur cette autre portion de manière à rendre l'extrémité 18a de la bande 4a solidaire de l'aile 8a.

La deuxième extrémité 20a libre de la bande 4a est susceptible d'être déplacée dans le plan A de la cale et en particulier d'être reliée à l'aile 10a symétrique de l'aile 8a par rapport au plan de symétrie C des gorges.

On va maintenant décrire les moyens de fixation auto-bloquants 14a et 16a de la bande 4a dans l'aile 10a sur lesquels la deuxième extrémité 20a est apte à être fixée. Par analogie, la description vaut également pour les moyens auto-bloquants ménagés dans l'aile 10b symétrique par rapport au plan de symétrie B de la cale.

Ces moyens de fixation de la cale sont constitués de deux percements 14a et 16a en forme de fentes traversant l'aile 10a de part en part. Ces fentes sont parallèles entre elles et également parallèles à la fente 12a ménagée dans la première aile 8a. De plus elles ont les mêmes dimensions que cette dernière fente 12a.

La fixation de la deuxième extrémité 20a de la bande se fait par l'insertion de cette extrémité dans la fente 16a depuis la paroi interne 24a de la gorge 6a. L'extrémité de la bande 20a est ensuite insérée dans la fente 14a sur la face opposée à la paroi de la gorge, pour déboucher dans cette dernière, puis tirée et appliquée contre la portion de bande qui pénètre dans la fente 16a. Ainsi la bande 4a constitue, en coopération avec la gorge 6a, une première boucle dans laquelle l'apophyse épineuse 26a est susceptible d'être engagée.

Par symétrie, les moyens de fixation précités sont identiques pour la gorge opposée 6b. De même, on procède de la même manière que décrit dessus pour constituer la seconde boucle.

Généralement les bandes 4a, 4b sont pré-montées sur la cale 2 de manière à insérer la cale 2 directement entre deux vertèbres et à enfiler les boucles formées directement sur les apophyses. Ensuite, le bout 28a de l'extrémité 20a est tiré pour serrer l'apophyse épineuse 26a entre la gorge 6a et la bande 4a de manière à bloquer la cale 2 sur l'apophyse 26a. Habituellement, la tension de la bande 4a dans les moyens de fixation auto-bloquants est suffisante pour assurer son blocage. En effet, les passage successifs de la bande 4a dans les deux fentes 16a et 14a induisent des forces de frottements importantes s'opposant au glissement de la bande 4a, notamment de par les arrêtes que présentent ces fentes.

De plus, la portion de bande qui débouche de la fente 14a sur la paroi interne 24a et la portion de bande qui pénètre dans la fente 16a sont comprimées entre la paroi interne 24a de la gorge 6a et l'apophyse épineuse 26a, ce qui accentue la force de blocage de l'extrémité 20a de la bande 4a sur l'aile 10a de la cale 2.

Les forces de frottements et la compression exercées sur l'extrémité 20a de la bande sont d'autant plus fortes que le serrage de ladite bande 4a est important. Cependant, dans le cas où le blocage est insuffisant, on prévoit de fixer le bout de l'extrémité 20a de la bande sur la portion de bande qui enserre l'apophyse 26a au moyen d'un clip 30a.

La cale 2 est immobilisée de façon identique sur l'apophyse épineuse 26b. Ainsi la cale 2 est fixée entre deux vertèbres sur les apophyses épineuses 26a, 26b de ces deux vertèbres.

En se référant maintenant à la Figure 2, on comprend que lorsque le rachis est en extension, la partie inférieure 32a de l'apophyse épineuse 26a et la partie supérieure 32b de l'apophyse épineuse 26b ont tendance à se rapprocher et qu'elles sont bloquées respectivement dans le fond des gorges 6a et 6b. Ainsi, en absence de disque intervertébral ou en présence d'un disque défectueux, la cale 2 limite le contact des deux vertèbres consécutives.

La cale 2 est généralement réalisée dans un matériau radio-transparent, de façon à la rendre invisible sur les clichés radiologiques pour ne pas masquer les organes que l'on souhaite pouvoir visualiser. Cependant, afin de situer la cale dans le rachis, on insère un élément radio-opaque transversal, suffisamment mince pour ne pas gêner l'observation des clichés, dans un logement central 34.

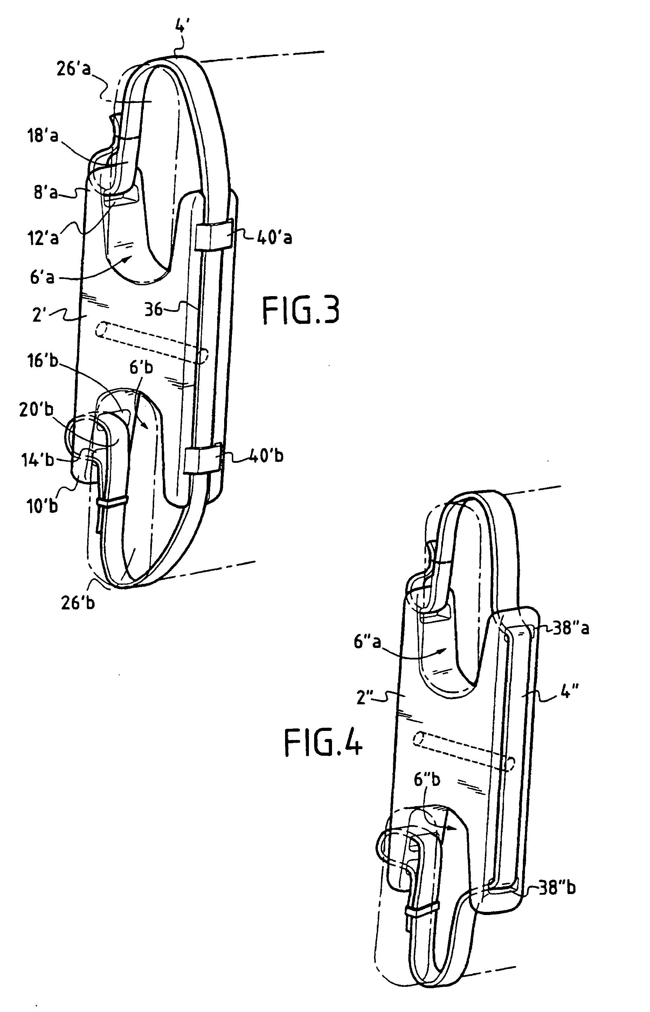

Sur la Figure 3, on a représenté une variante de réalisation de l'implant intervertébral dans laquelle ledit lien comporte une bande 4 dont la première extrémité 18'a est fixée auxdits moyens de fixation 12'a ménagés dans une première aile 8'a de la première gorge 6'a et dont la deuxième extrémité 20'b est fixée auxdits moyens de fixation auto-bloquant 16'b, 14'b ménagés dans la seconde aile 10'b de la deuxième gorge 6'b, symétrique de la première aile 8'a de la première gorge 6'a par rapport à ladite cale 2',
ladite cale comprenant en outre des moyens de guidage ménagés dans ladite cale 2' pour guider ladite bande 4' dans le prolongement de la seconde aile de la première gorge 6'a et la première aile de la seconde gorge 6'b par quoi lesdites apophyses 26'a, 26'b sont enserrées simultanément.

Dans cette variante de réalisation les moyens de guidage comprennent une rainure rectiligne 36, pratiquée sur la paroi externe de ladite cale 2' dans le prolongement de la seconde aile de la première gorge 6'a et de la première aile de la seconde gorge 6'b, dans laquelle ladite bande 4' est apte à coulisser. Afin de maintenir la bande 4' dans la rainure 36 on prévoit des cavaliers 40'a et 40'b situés au-dessus de la rainure 36 au niveau des ailes.

Ce mode de réalisation permet avantageusement de serrer simultanément les deux apophyses épineuses dans les gorges 6'a et 6'b par une seule action sur la deuxième extrémité de la bande 4'.

Sur la Figure 4 on a représenté une variante de réalisation de l'implant intervertébral comportant une seule bande, dans laquelle les moyens de guidage comprennent une fente 38"a ménagée dans la seconde aile de la première gorge 6"a et une fente 38"b dans la première aile de la seconde gorge 6"b.

## Revendications

1. Implant intervertébral comprenant une cale (2) dans laquelle sont ménagées deux gorges (6a, 6b) opposées susceptibles de recevoir les deux apophyses épineuses (26a, 26b) de deux vertèbres, chaque gorge définissant deux ailes (8a, 10a, 8b, 10b) ayant une paroi interne, et un lien (4a, 4b) pour maintenir ladite cale sur lesdites apophyses épineuses,
- ledit lien étant constitué d'au moins une bande dont une partie peut entourer une portion de surface de l'apophyse opposée au fond dé la gorge,
ledit implant comprenant en outre
- des moyens de fixation (12a, 12b), ménagés dans au moins une desdites ailes (8a, 8b), pour fixer une première extrémité (18a, 18b) de ladite bande, et
**caractérisé en ce que** ledit implant comprend :
- des moyens de fixation auto-bloquants (14a, 16a, 14b, 16b), ménagés dans au moins une autre desdites ailes (10a, 10b), au travers desquels la deuxième extrémité (20a, 20b) de ladite bande peut être insérée puis tirée pour maintenir en position ladite bande (4a, 4b) par quoi ladite cale (2) est solidaire desdites apophyses épineuses (26a, 26b).

2. Implant intervertébral selon la revendication 1, **caractérisé en ce que** les moyens de fixation (12a, 12b) comprennent une fente, percée dans ladite aile (8a, 8b), dans laquelle ladite première extrémité (18a, 18b) de ladite bande (4a, 4b) est susceptible d'être engagée de manière à former une boucle, par quoi ladite première extrémité (18a, 18b) de ladite bande (4a, 4b) est solidaire de ladite aile (8a, 8b).

3. Implant intervertébral selon la revendication 1 ou 2, **caractérisé en ce que** les moyens de fixation auto-bloquants (14a, 16a, 14b, 16b) comprennent une première fente (14a, 14b) et une deuxième fente (16a, 16b) parallèles entre elles, percées dans ladite autre aile (10a, 10b), ladite deuxième fente (16a, 16b) étant située entre ladite première fente (14a, 14b) et le fond de la gorge (6a, 6b), de sorte que la portion de bande qui débouche de la première fente sur ladite paroi interne de ladite autre aile est appliquée contre la portion de bande qui pénètre dans la deuxième fente par quoi l'extrémité de ladite bande est susceptible d'être bloquée par frottement.

4. Implant intervertébral selon l'une quelconque des revendications 1 à 3, **caractérisé en ce que** chaque gorge (6a, 6b) comprend des moyens de fixation (12a, 12b) dans l'une de ses ailes (8a, 8b) et des moyens de fixations auto-bloquants (14a, 16a, 14b, 16b) ménagés dans l'autre aile (10a, 10b), de manière à fixer deux bandes (4a, 4b), chacune étant apte à entourer chacune des apophyses (26a, 26b), par quoi lesdites apophyses (26a, 26b) sont enserrées indépendamment l'une de l'autre.

5. Implant intervertébral selon l'une quelconque des revendications 1 à 3, **caractérisé en ce qu'**une première aile (18a) de la première gorge (6'a) comprend des moyens de fixation (12'a, 12'b) pour fixer la première extrémité d'une bande (18a),
**en ce que** la seconde aile (10'b) de la deuxième gorge (6'b), symétrique de la première aile de la première gorge (6'a) par rapport à ladite cale (2'), comprend des moyens de fixation auto-bloquants (14'b, 16'b) pour bloquer la seconde extrémité (20'b) de ladite bande,
et **en ce que** ladite cale (2') comprend en outre des moyens de guidage ménagés dans ladite cale (2') pour guider ladite bande (4') dans le prolongement de la seconde aile de la première gorge (6'a) et la première aile de la seconde gorge (6'b) par quoi lesdites apophyses (26a, 26b) sont enserrées simultanément

6. Implant intervertébral selon la revendication 5, **caractérisé en ce que** les moyens de guidage comprennent une fente (38"a) ménagée dans la seconde aile de la première gorge (6"a) et une fente (38"b) dans la première aile de la seconde gorge (6"b).

7. Implant intervertébral selon la revendication 5, **caractérisé en ce que** les moyens de guidage comprennent une rainure rectiligne (36'), pratiquée sur la paroi externe de ladite cale (2') dans le prolongement de la seconde aile de la première gorge (6'a) et de la première aile de la seconde gorge (6'b), dans laquelle ladite bande (4') est apte à coulisser.

## Claims

1. An intervertebral implant comprising a spacer (2) in which two opposite notches (6a, 6b) are formed suitable for receiving the two spinous processes (26a, 26b) of two vertebrae, each notch defining two flanges (8a, 10a, 8b, 10b) each having an inside wall, the implant also having a tie (4a, 4b) for holding said spacer to said spinous processes;
- said tie being constituted by at least one strap having a portion that can surround a portion of the surface of the spinous process opposite the bottom of the notch;
said implant further comprising
- fixing means (12a, 12b) formed in at least one of said flanges (8a, 8b) to fix a first end (18a, 18b) of said strap; and
**characterised in that** said implant comprises:
- self-locking fixing means (14a, 16a, 14b, 16b) formed in at least one other of said flanges (10a, 10b), the second end (20a, 20b) of said strap may be inserted through said self-locking fixing means and then pulled to hold said strap (4a, 4b) in position, thereby securing said spacer (2) to said spinous processes (26a, 26b).

2. An intervertebral implant according to claim 1, **characterised in that** the fixing means (12a, 12b) comprise a slot formed through said flange (8a, 8b), said first end (18a, 18b) of said strap (4a, 4b) being suitable for being engaged in said slot in such a manner as to form a loop whereby said first end (18a, 18b) of said strap (4a, 4b) is secured to said flange (8a, 8b).

3. An intervertebral implant according to claim 1 or 2, **characterised in that** the self-locking fixing means (14a, 16a, 14b, 16b) comprise a first slot (14a, 14b) and a second slot (16a, 16b) that are parallel to each other, said slots being formed through the other flange (10a, 10b), said second slot (16a, 16b) being situated between said first slot (14a, 14b) and the bottom of the notch (6a, 6b) such that the portion of the strap which passes through the first slot and projects from said inside wall of said other flange is pressed against the portion of the strap which penetrates into the second slot, thereby enabling the end of said strap to be locked by friction.

4. An intervertebral implant according to any one of claims 1 to 3, **characterised in that** each notch (6a, 6b) has first fixing means (12a, 12b) in one of its flanges (8a, 8b) and self-locking fixing means (14a, 16a, 14b, 16b) formed in its other flange (10a, 10b) so as to enable two straps (4a, 4b) to be fixed, each strap being suitable for passing round a respective one of the spinous processes (26a, 26b), whereby said spinous processes (26a, 26b) are held independently of each other.

5. An intervertebral implant according to any one of claims 1 to 3, **characterised in that** a first flange (18a) of the first notch (6'a) has fixing means (12'a, 12'b) for fixing the first end of a strap (18a);
**in that** the second flange (10'b) of the second notch (6'b) symmetrical to the first flange of the first notch (6'a) about said spacer (2') has self-locking fixing means (14'b, 16'b) for locking the second end (20'b) of said strap; and
**in that** said spacer (2') also includes guide means formed in said spacer (2') to guide said strap (4') in line with the second flange of the first notch (6'a) and the first flange of the second notch (6'b) whereby said spinous processes (26a, 26b) are tightened simultaneously.

6. An intervertebral implant according to claim 5, **characterised in that** the guide means comprise a slot (38"a) formed through the second flange of the first notch (6'a) and a slot (38"b) formed through the first flange of the second notch (6"b).

7. An intervertebral implant according to claim 5, **characterised in that** the guide means comprise a rectilinear groove (36") formed in the outside wall of said spacer (2') in line with the second flange of the first notch (6'a) and with the first flange of the second notch (6'b), and in which said strap (4') is suitable for sliding.

## Patentansprüche

1. Zwischenwirbelimplantat mit einem Keil (2), in dem zwei Hohlkehlen (6a, 6b) einander gegenüberliegend angeordnet sind, die die zwei Wirbelfortsätze (26a, 26b) von zwei Wirbeln aufnehmen können, wobei jede Hohlkehle zwei Flügel (8a, 10a, 8b, 10b) definiert, die eine Innenwand und eine Verbindung (4a, 4b) haben, um den Keil auf den Wirbelfortsätzen zu halten,
- wobei besagte Verbindung aus mindestens einem Band besteht, von dem ein Teil einen Abschnitt der Oberfläche des dem Boden der Hohlkehlen gegenüberliegenden Wirbelfortsatzes umgeben kann,
wobei das Implantat ferner
- Befestigungsmittel (12a, 12b) umfaßt, die in mindestens einem der Flügel (8a, 8b) angeordnet sind, um ein erstes Ende (18a, 18b) des Bandes zu befestigen,
**dadurch gekennzeichnet, daß** das Implantat folgendes umfaßt:
- selbstblockierende Befestigungsmittel (14a, 16a, 14b, 16b), die in mindestens einem anderen der Flügel (10a, 10b) angeordnet sind, durch welche das zweite Ende (20a, 20b) des Bands eingefügt und dann gezogen werden kann, um das Band (4a, 4b) in Position zu halten, wodurch der Keil (2) fest mit den Wirbelfortsätzen (26a, 28b) verbunden ist.

2. Zwischenwirbelimplantat nach Anspruch 1, **dadurch gekennzeichnet, daß** die Befestigungsmittel (12a, 12b) einen Schlitz umfassen, der in den Flügel (8a, 8b) gebohrt ist, in den das erste Ende (18a, 18b) des Bands (4a, 4b) so eingefügt werden kann, daß es eine Schleife bildet, wodurch das erste Ende (18a, 18b) des Bandes (4a, 4b) mit dem Flügel (8a, 8b) fest verbunden ist.

3. Zwischenwirbelimplantat nach Anspruch 1 oder 2, **dadurch gekennzeichnet, daß** die selbstblockierenden Befestigungsmittel (14a, 16a, 14b, 16b) einen ersten Schlitz (14a, 14b) und einen zweiten Schlitz (16a, 16b), die zueinander parallel und die in den anderen Flügel (10a, 10b) gebohrt sind, umfassen, wobei sich der zweite Schlitz (16a, 16b) zwischen dem ersten Schlitz (14a, 14b) und dem Grund der Hohlkehle (6a, 6b) befindet, so daß der Abschnitt des Bandes, der von dem ersten Schlitz auf die Innenwand des anderen Flügels mündet, gegen den Abschnitt des Bandes gedrückt Bandes gedrückt wird, der in den zweiten Schlitz eindringt, wodurch das Ende des Bandes durch Reibung blockiert werden kann.

4. Zwischenwirbelimplantat nach einem der Ansprüche 1 bis 3, **dadurch gekennzeichnet, daß** jede Hohlkehle (6a, 6b) Befestigungsmittel (12a, 12b) in einem ihrer Flügel (8a, 8b) umfaßt sowie selbstblockierende Befestigungsmittel (14a, 16a, 14b, 16b), die in dem anderen Flügel (10a, 10b) so angeordnet sind, daß zwei Bänder (4a, 4b) befestigt werden, wobei jedes jeden der Wirbelfortsätze (26a, 26b) umgeben kann, wodurch die Wirbelfortsätze (26a, 26b) unabhängig voneinander eingeschlossen sind.

5. Zwischenwirbelimplantat nach einem der Ansprüche 1 bis 3, **dadurch gekennzeichnet,**
**daß** ein erster Flügel (18a) der ersten Hohlkehle (6'a) Befestigungsmittel (12'a, 12'b) umfaßt, um das erste Ende eines Bands (18a) zu befestigen,
**daß** der zweite Flügel (10'b) der zweiten Hohlkehle (6'b), der zum ersten Flügel der Hohlkehle (6'a) in Bezug auf den Keil (2') symmetrisch ist, selbstblockierende Befestigungsmittel (14'b, 16'b) umfaßt, um das zweite Ende (20'b) des Bands zu blockieren, und
**daß** der Keil (2') ferner Führungsmittel umfaßt, die in dem Keil (2') angeordnet sind, um das Band (4') in der Verlängerung des zweiten Flügels der ersten Hohlkehle (6'a) und den ersten Flügel der zweiten Hohlkehle (6'b) zu führen, wodurch die Wirbelfortsätze (26a, 26b) gleichzeitig eingeschlossen werden.

6. Zwischenwirbelimplantat nach Anspruch 5, **dadurch gekennzeichnet, daß** die Führungsmittel einen Schlitz (38"a) umfassen, der in dem zweiten Flügel der ersten Hohlkehle (6"a) angeordnet ist, und einen Schlitz (38"b) in dem ersten Flügel der zweiten Hohlkehle (6"b).

7. Zwischenwirbelimplantat nach Anspruch 5, **dadurch gekennzeichnet, daß** die Führungsmittel eine geradlinige Rille (36') umfassen, die an der Außenwand des Keils (2') in der Verlängerung des zweiten Flügels der ersten Hohlkehle (6'a) und des ersten Flügels der zweiten Hohlkehle (6'b) angebracht ist, in der das Band (4') gleiten kann.
